# EUROPÄISCHE PATENTSCHRIFT

(11) **EP 0 347 743 B1**
(45) Veröffentlichungstag und Bekanntmachung des Hinweises auf die Patenterteilung: **28.04.1993**
(21) Anmeldenummer: 89110846.6
(22) Anmeldetag: 15.06.1989
(51) Int. Cl.: A61M 5/14

(54) **Septum für implantierbare Vorrichtungen zur Abgabe von Wirkstoffen**
Septum for implantable devices releasing agents
Septum pour dispositifs implantables libérant des agents

(30) Priorität: 23.06.1988 AT 1631/88
(43) Veröffentlichungstag der Anmeldung: 27.12.1989
(73) Patentinhaber: Fresenius AG, 61350 Bad Homburg v.d.H (DE)
(72) Erfinder:
(74) Vertreter: Fuchs Mehler Weiss

(56) Entgegenhaltungen:
- DE-A- 3 515 624
- GB-A- 2 121 690
- US-A- 4 193 397

## Beschreibung

Die Erfindung bezieht sich auf eine Anordnung eines Septums in einer implantierbaren Vorrichtung zur Abgabe von Wirkstoffen, z.B. Medikamenten, an den Körper des Patienten, mit einem Septum und einer Vorrichtung für dessen Befestigung am Gehäuse einer Medikamentenkammer wobei das Septum als Abdeckung der Medikamentenkammer in die zugehörige Öffnung deren Gehäuses durch ein oder mehrere Klemmorgane dicht eingesetzt ist. Solche Vorrichtungen zeigen beispielsweise die GB-A-2 121 690 oder die US-A-4 193 397.

Bei bekannten Ausbildungen dieser Art ist bisher ein kreisrundes Septum verwendet worden, das am Rand durch Klemmorgane in der Vorrichtung gehalten ist. Der Mittelbereich des Septums hat die Öffnung der Medikamentenkammer frei überspannt, so daß der Größe des Septums durch die Spannweite insofern Grenzen gesetzt sind, als das Septum ein sehr elastischer, gummiartiger Körper ist, der sich nach jedem Einstich einer Injektionsnadel wieder dicht verschließen muß, wodurch bei zu großer Spannweite das Septum beim Einstechen am Boden der Kammer zur Anlage kommt, wodurch das Nachfüllen der Kammer erheblich behindert wird. Aufgrund der bei den bekannten Ausbildungen nur kleinen zur Verfügung stehenden Fläche zum Einstechen mittels der Injektionsnadel wird jede Einstichstelle sehr oft durch die Injektionsnadel benützt, so daß die bisherigen Septen bald zum Undichtwerden neigen.

Der Erfindung liegt die Aufgabe zugrunde, ein Septum der eingangs genannten Art zu schaffen, bei welchem eine größere Fläche zum Einstechen zur Verfügung steht, ohne daß die freie Spannweite des Septums verändert wird.

Erfindungsgemäß wird diese Aufgabe dadurch gelöst, daß das Septum ringförmig ausgebildet ist, wobei die längs der Innenkante des Septums angreifenden Klemmorgane mit einem mit dem übrigen Gehäuse fest verbundenen Teil der Vorrichtungswandung zusammenwirken. Dadurch wird erreicht, daß eine wesentlich größere Fläche für das Einstechen der Injektionsnadel zur Verfügung steht, so daß das Septum durch die Einstiche der Injektionsnadel nicht so beansprucht wird. Durch die erfindungsgemäße Ausbildung wird zwar die Vorrichtung zur Abgabe von Wirkstoffen durchmessermäßig etwas größer, jedoch ist dieser Nachteil durch die wesentlich längere Haltbarkeit des Septums bei weitem aufgehoben.

In besonders vorteilhafter Weise eignet sich das erfindungsgemäße Septum für implantierbare Vorrichtungen zur dosierten Abgabe eines Stoffes, z.B. Medikaments, wobei die Vorrichtung zwei voneinander durch eine flexible Membran getrennte Kammern aufweist, von welchen die eine Kammer, die den abzugebenden Stoff enthält, mit einer mit einem Ausgabekatheter verbundenen Ausgabeöffnung und einer Nachfüllöffnung versehen ist, und die andere Kammer mit einem Treibmittel gefüllt ist, das sich zur Ausgabe des abzugebenden Stoffes unter Verdampfung isobar ausdehnt. Bei einer solchen Ausbildung kann das erfindungsgemäße Septum zur Abdichtung der Kammer, die den abzugebenden Stoff enthält, dienen. Auch dadurch kann erreicht werden, daß die Nachfüllung der Kammer wesentlich öfter durchgeführt werden kann, ohne daß das Septum undicht wird.

Weiters ist das anmeldungsgemäße Septum auch an einer implantierbaren Vorrichtung zur dosierten Abgabe eines Stoffes, z.B. Medikaments, vorteilhaft, bei welcher die Vorrichtung zwei voneinander durch eine flexible Membran getrennte Kammern aufweist, von welchen die eine Kammer, die den abzugebenden Stoff enthält, mit einer mit einem Ausgabekatheter verbundenen Ausgabeöffnung und einer Nachfüllöffnung versehen ist, und die andere Kammer mit einem Treibmittel gefüllt ist, das sich zur Ausgabe des abzugebenden Stoffes unter Verdampfung isobar ausdehnt, wobei zwischen der Ausgabeöffnung der Kammer und dem Ausgabekatheter eine Einrichtung für ein zusätzliches Eingeben von Medikamenten od. dgl. in den Auslaßkatheter vorgesehen ist. Bei einer solchen Vorrichtung kann das ringförmige Septum zur Abdichtung der Kammer für das zusätzliche Eingeben von Medikamenten dienen, wobei das Septum um die Nachfüllöffnung herum angeordnet ist. Dadurch ist neben der Vergrößerung der Oberfläche, die von der Injektionsnadel zu durchdringen ist, erreicht, daß das Septum leichter aufgefunden werden kann. Dieses Auffinden der ringförmigen Kammer kann überdies auch noch durch eine Schablone wesentlich erleichtert werden, die auf die Haut des Patienten gelegt wird, wobei die Lage der Schablone etwa durch den tastbaren Rand der implantierten Vorrichtung bestimmt wird. Schließlich kann die Nachfüllöffnung zentrisch an der Vorrichtung vorgesehen und das ringförmige Septum konzentrisch zur Nachfüllöffnung angeordnet sein. Dadurch kann zum Auffinden des ringförmigen Septums die Schablone auch so auf der implantierten Vorrichtung aufgesetzt werden, daß das Zentrum der Schablone mit der zentralen, nach außen vorspringenden Nachfüllöffnung übereinstimmt. Die Schablone besitzt dann in einer dem Radius der ringförmigen Kammer entsprechenden Entfernung vom Zentrum eine durchgehende Öffnung, welche den Ort der ringförmigen Kammer anzeigt. Wenn die Lage der Schablone durch den tastbaren Rand der implantierten Vorrichtung bestimmt wird, dann muß die Nachfüllöffnung genau zentrisch in der Vorrichtung angeordnet sein. Zur Bestimmung des Ortes der ringförmigen Kammer könnte auch der Rand der Schablone dienen, wenn deren Durchmesser jenem der ringförmigen Kammer entspricht.

Derartige implantierbare Vorrichtungen zur dosierten Abgabe eines Stoffes dienen üblicherweise dazu, Insulin oder sonstige Dauermedikamente in den Körper direkt einzubringen, wobei diese Vorrichtungen über die Nachfüllöffnungen, welche durch ein Septum abgeschlossen sind, mittels einer Injektionsspritze nachgefüllt werden. Nun ist es aber vielfach erforderlich, daß zu der kontinuierlichen Dosierung des Medikaments auch zusätzliches Medikament gegeben werden muß. Dazu ist es bereits bekannt, eine zusätzliche Kammer vorzusehen, welche mit dem Auslaßkatheter in Verbindung steht, und über welche zusätzliches Medikament direkt in den Auslaßkatheter eingespritzt werden kann. Diese Kammer ist bei der bekannten Ausführung an der Außenperipherie der Vorrichtung vorgesehen, und ebenso wie die Nachfüllöffnung mit einem kreisrunden Septum abgeschlossen, durch welches hindurch mittels einer Injektionsnadel das zusätzliche Einspritzen des Medikaments ermöglicht ist. Diese bekannte Ausführung hat den Nachteil, daß der Arzt, der das zusätzliche Medikament einspritzt, diese zusätzlich mit einem Septum verschlossene Öffnung der Kammer erst suchen muß, was bei einer implantierten Vorrichtung nicht ganz einfach ist. Außerdem wird die Stelle zur zusätzlichen Einführung des Medikaments von der Injektionsnadel sehr oft durchstochen, was dann in der Folge zu Undichtheiten des Septums führen kann. All diese genannten Nachteile werden durch die vorstehend beschriebene erfindungsgemäße Ausbildung vermieden.

In der Zeichnung sind einige Ausführungsbeispiele des Erfindungsgegenstandes dargestellt.
Fig. 1 zeigt einen Vertikalschnitt durch eine mit einem erfindungsgemäßen Septum versehene Vorrichtung zur dosierten Abgabe eines Medikaments, und zwar nach Linie I-I der Fig. 2.
Fig. 2 veranschaulicht eine Draufsicht auf die erfindungsgemäße Vorrichtung gemäß Anspruch 1.
Fig. 3 zeigt einen Vertikalschnitt durch eine mit dem erfindungsgemäßen Septum versehene Zuspritzeinrichtung für ein Medikament.
Fig. 4 zeigt einen Schnitt nach Linie IV-IV der Fig. 5 einer anderen Ausführungsform einer Zuspritzeinrichtung, welche gleichfalls mit dem erfindungsgemäßen Septum versehen ist.
Fig. 5 zeigt einen Schnitt nach Linie V-V der Fig. 4.

Die Vorrichtung weist ein aus zwei Teilen 1, 2 zusammengesetztes Gehäuse auf, dessen Innenraum durch eine flexible Membran 3 in zwei Kammern 4, 5 geteilt ist. Die Kammer 4 ist dabei zur Aufnahme des abzugebenden Medikaments bestimmt, wobei die Kammer 5 ein Treibmittel enthält, welches sich durch die Körperwärme isobar ausdehnt. Durch das Ausdehnen des Treibmittels wird die Membran 3 im Sinne einer Verdrängung des Medikaments aus der Kammer 4 beaufschlagt, wobei das Medikament über die Auslaßöffnung 6, eine Auslaßreduziereinrichtung 7 und einen Auslaßkatheter 8 in den Körper des Patienten abgegeben wird. Bevor das Medikament den Auslaßkatheter 8 erreicht, wird es in eine Kammer 9 eingeleitet, welche ringförmig am dem Teil 1 des Gehäuses vorgesehen ist. Diese Kammer 9 ist an ihrer Oberseite durch einen Ring 10, welcher von einer Injektionsnadel durchstochen werden kann und sich nach Herausziehen der Injektionsnadel selbsttätig wieder abdichtet, also durch ein Septum, abgeschlossen. Dieser Ring 10 wird über eine ringförmige Befestigungseinrichtung 11 in seiner Lage festgehalten. Die Kammer 4 ist über ein weiteres Septum 12, welches mittels eines Befestigungsteiles 13 festgehalten ist, nachfüllbar.

Vorliegend ist die implantierbare Vorrichtung als Rotationskörper ausgebildet, wobei die Nachfüllöffnung 12 in der Vorrichtung zentrisch und die ringförmige Kammer 9 konzentrisch dazu angeordnet ist. Zur Auffindung der ringförmigen Kammer 9 ist eine - nicht dargestellte - Schablone so auf die Haut des Patienten auflegbar, daß das Zentrum der Schablone mit der Nachfüllöffnung übereinstimmt. Die Schablone besitzt in einem, dem Radius der ringförmigen Kammer entsprechenden Abstand vom Zentrum ein Loch, welches sich dann nach entsprechendem Auflegen der Schablone über der ringförmigen Kammer befindet, so daß bei Einstechen der Injektionsnadel durch das Loch hindurch zuverlässig die ringförmige Kammer aufgefunden wird.

Die Auslaßreduziereinrichtung 7 ist im vorliegenden Fall durch eine Schlauchwindung gebildet, welche mehrfach in einer Ausnehmung des Teiles 1 des Gehäuses herumgewunden ist. Diese Ausnehmung ist durch einen Deckel 14 so abgedichtet, daß eine glatte äußere Kontur der implantierbaren Vorrichtung erzielt ist.

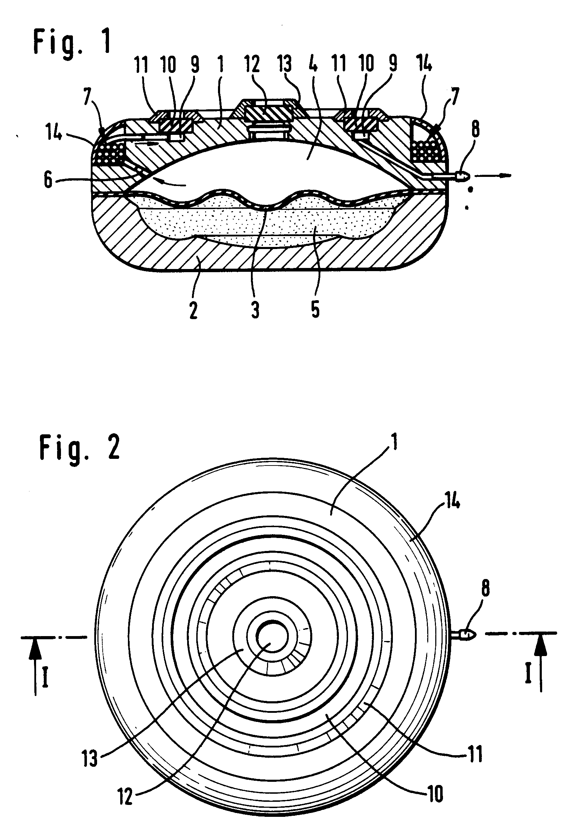

Wie aus Fig. 2 hervorgeht, ist die Kammer 9 und damit der Abdichtring 10 konzentrisch um die Nachfüllöffnung 12 herum angeordnet.

Das implantierte Gerät gibt über den Auslaßkatheter 8 kontinuierlich das in der Kammer 4 befindliche Medikament an den Körper des Patienten ab. Soll nun aus gesundheitlichen Gründen mehr von diesem Medikament oder ein anderes zusätzliches Medikament an den Körper des Patienten abgegeben werden, dann wird mittels einer Injektionsspritze, der der Einstichort von der aufgesetzten Schablone gewiesen wird, der Ring 10 durchstochen und das abzugebende Medikament in die Kammer 9 injiziert, von welcher es unmittelbar über den Auslaßkatheter 8 an den Körper abgegeben wird. Der Flußwiderstand in der Auslaßreduziereinrichtung 7 ist dabei so groß, daß ein Zurückdrängen des Medikaments in die Kammer 4 der implantierbaren Vorrichtung verhindert ist. Es muß sich dabei nicht unbedingt um dasselbe Medikament handeln, das über die Kammer 4 ausgegeben wird, sondern es kann z.B. auch zur Freimachung des Katheters 8 Heparin oder eine ähnliche Substanz eingespritzt werden.

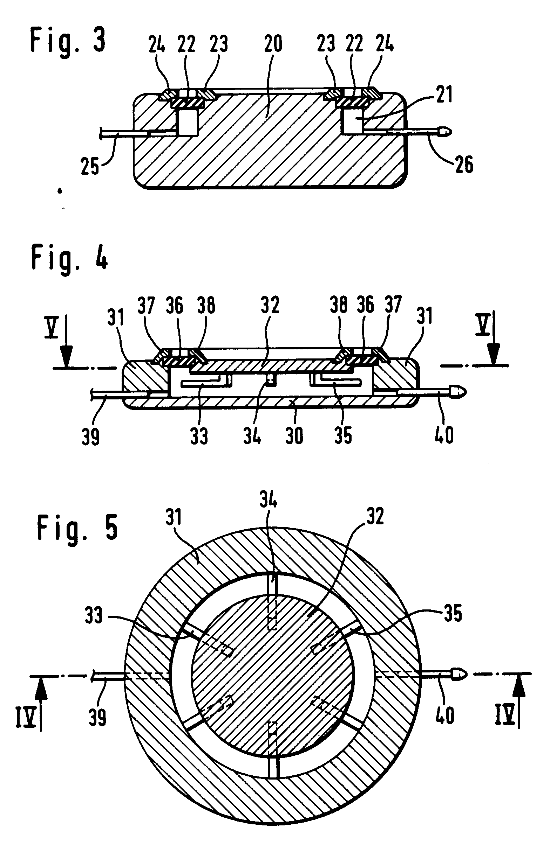

Beim Ausführungsbeispiel nach Fig. 3 ist in einem Gehäuse 20 eine ringförmige Kammer 21 vorgesehen, welche nach oben, also in Richtung zur Stirnfläche des Gehäuses 20 offen ist. Dort ist die Kammer 21 mittels eines ringförmigen Septums 22 abgeschlossen, welches mittels ringförmiger Klemmorgane 23, 24 im Gehäuse 20 festgelegt ist. Mit 25 ist die Zuleitung für eine Dauermedikament und mit 26 der Abgabekatheter bezeichnet.

Bei der Ausführungsform gemäß Fig. 4 ist gleichfalls ein scheibenförmiges Gehäuse vorgesehen, welches aus zwei Teilen besteht, nämlich aus dem Boden 30 und einem Oberteil 31. In diesem Oberteil 31 ist eine kreisförmige Öffnung vorgesehen, in welche zentral ein Mittelstück 32 eingesetzt ist, welches über Arme 33, 34, 35 am Oberteil festgelegt ist. Zwischen dem Oberteil 31 und dem Mittelstück 32 ist ein ringförmiger Raum freigelassen, welcher über ein gleichfalls ringförmiges Septum 36 abgeschlossen ist. Das ringförmige Septum 36 ist wieder mit ringförmigen Klemmorganen 37, 38 an dem Oberteil bzw. am Mittelstück befestigt. Das Mittelstück 32 weist dabei geringere Dicke auf als der Oberteil 31 hoch ist, so daß im Inneren des Gehäuses eine Kammer freigelassen ist, in welcher sich das eingespritzte Medikament befindet. Mit 39 ist die Zuleitung und mit 40 der Auslaßkatheter bezeichnet. Die Form der Kammer ist dabei unkritisch und kann entsprechend dem Einsatzzweck gewählt werden.

Bei den Ausführungsformen gemäß Fig. 3 bis 5 handelt es sich um bloße Zuspritzstellen von Medikament, wogegen die Ausführungsform nach Fig. 1 und 2 eine Vorrichtung zur dosierten Dauerabgabe von Medikamenten ist, welche zusätzlich eine Einrichtung zur Zuspritzung von Medikamenten aufweist. All den Ausführungsformen ist gemeinsam, daß das Septum bzw. eines der Septen ringförmig ausgebildet ist, wodurch die vorstehend dargelegten Vorteile erzielt werden.

In Abwandlung der in den Fig. 3 bis 5 dargestellten Ausbildungen, welche als Zusatz zu einer Infusionspumpe einsetzbar sind, kann die erfindungsgemäße Ausbildung auch als bloße Einspritzstelle für Medikamente dienen, die zu speziellen Organen zu bringen sind, wobei das diesbezügliche Hohlorgan, wie z.B. die zubringende Vene, nicht oder nur sehr schwer zugänglich ist. In diesem Fall entfallen dann die Zuleitungen 25 bzw. 39, so daß von der implantierten Einrichtung lediglich der Auslaßkatheter 26 bzw. 40 wegführt.

## Patentansprüche

1. Anordnung eines Septums in einer implantierbaren Vorrichtung zur Abgabe von Wirkstoffen, z.B. Medikamenten, an den Körper des Patienten, mit einem Septum und einer Vorrichtung für dessen Befestigung am Gehäuse einer Medikamentenkammer wobei das Septum (10; 22; 36) als Abdeckung der Medikamentkammer (9; 21) in die zugehörige Öffnung deren Gehäuses durch ein oder mehrere Klemmorgane (23, 24; 37, 38; 11) dicht eingesetzt ist, dadurch gekennzeichnet, daß das Septum (22; 36; 10) ringförmig ausgebildet ist, wobei die längs der Innenkante des Septums angreifenden Klemmorgane mit einem mit dem übrigen Gehäuse (31) fest verbundenen Teil (20; 32; 1) der Vorrichtungswandung zusammenwirken.

2. Mit einem Septum gemäß Anspruch 1 versehene Vorrichtung zur dosierten Abgabe eines Stoffes, z.B. Medikaments, wobei die Vorrichtung zwei voneinander durch eine flexible Membran getrennte Kammern aufweist, von welchen die eine Kammer, die den abzugebenden Stoff enthält, mit einer mit einem Ausgabekatheter verbundenen Ausgabeöffnung und einer Nachfüllöffnung versehen ist, und die andere Kammer mit einem Treibmittel gefüllt ist, das sich zur Ausgabe des abzugebenden Stoffes unter Verdampfung isobar ausdehnt, dadurch gekennzeichnet, daß das Septum (10) zur Abdichtung der Kammer (9), die den abzugebenden Stoff enthält, dient.

3. Mit einem Septum gemäß Anspruch 1 versehene Vorrichtung zur dosierten Abgabe eines Stoffes, z.B. Medikaments, wobei die Vorrichtung zwei voneinander durch eine flexible Membran getrennte Kammern aufweist, von welchen die eine Kammer, die den abzugebenden Stoff enthält, mit einer mit einem Ausgabekatheter verbundenen Ausgabeöffnung und einer Nachfüllöffnung versehen ist, und die andere Kammer mit einem Treibmittel gefüllt ist, das sich zur Ausgabe des abzugebenden Stoffes unter Verdampfung isobar ausdehnt, wobei zwischen der Ausgabeöffnung der Kammer und dem Ausgabekatheter eine Einrichtung für ein zusätzliches Eingeben von Medikamenten od. dgl. in den Auslaßkatheter vorgesehen ist, dadurch gekennzeichnet, daß das ringförmige Septum (10) zur Abdichtung der Kammer (9) für das zusätzliche Eingeben von Medikamenten dient, wobei das Septum um die Nachfüllöffnung (12) herum angeordnet ist.

4. Mit einem Septum versehene Vorrichtung nach Anspruch 3, dadurch gekennzeichnet, daß die Nachfüllöffnung (12) zentrisch an der Vorrichtung vorgesehen und das ringförmige Septum (10) konzentrisch zur Nachfüllöffnung (12) angeordnet ist.

## Claims

1. Arrangement of a septum in an implantable device for dispensing active substances, for example medicaments, to the body of the patient, said arrangement comprising a septum and a device for its bonding to the housing of a medicament chamber, said septum (10; 22, 36) being tightly inserted as cover of said medicament chamber (9; 21) into the associated opening via one or more clamping members (23, 24; 37, 38; 11), characterized in that the septum (22; 36; 10) is embodied annularly, whereby the clamping members abutting along the inner edge of the septum correspond with a portion (20; 32; 1) of the device wall being fixedly connected to the remaining housing (31).

2. Device provided with the septum according to claim 1 for dosed dispensing of a substance, for example a medicament, the device having two chambers separated from each other by a flexible membrane, one of which chambers containing the substance to be dispensed, and having a dispensing opening connected to a dispensing catheter and a replenishing opening, and the other chamber being filled with a propellant which expands isobarically with evaporation to dispense the substance to be liberated, characterized in that the septum (10) serves to seal the chamber (9) containing the substance to be dispensed.

3. Device provided with the septum according to claim 1 for dosed dispensing of a substance, for example a medicament, the device having two chambers separated from each other by a flexible membrane, one of which chambers containing the substance to be dispensed, and having a dispensing opening connected to a dispensing catheter and a replenishing opening, and the other chamber being filled with a propellant which expands isobarically with evaporation to dispense the substance to be liberated, whereby between the dispensing opening of the chamber and the dispensing catheter a means is provided for an additional introduction of medicaments or the like into the outlet catheter, characterized in that the annular septum (10) serves for sealing the chamber (9) for the additional introduction of medicaments, the septum being arranged around the replenishing opening (12).

4. Device provided with a septum according to claim 3, characterized in that the replenishing opening (12) is arranged centrally at the device and the annular septum (10) is arranged concentrically to the replenishing opening (12).

## Revendications

1. Dispositif comprenant un septum dans un dispositif implantable servant à délivrer des substances actives, par exemple des médicaments, dans le corps d'un patient, comportant un septum et un dispositif pour la fixation de ce septum au boîtier d'une chambre à médicament, le septum (10;22;36) étant inséré en tant que fermeture de la chambre à médicament (9;21), dans l'ouverture associée du boîtier de cette chambre, d'une manière étanche dans le cas de la présence d'un ou de plusieurs organes de serrage (23,24;37;38;11), caractérisé par le fait que le septum (22;36;10) possède une forme annulaire, les organes de serrage, qui s'accrochent le long du bord intérieur du septum, coopérant avec une partie (20;32;1) de la paroi du dispositif, qui est raccordée fermement au reste du boîtier (31).

2. Dispositif équipé d'un septum selon la revendication 1, pour la délivrance dosée d'une substance, par exemple d'un médicament, le dispositif possédant deux chambres séparées l'une de l'autre par une membrane flexible et dont l'une, qui contient la substance devant être délivrée, est pourvue d'une ouverture de sortie raccordée à un cathéter de sortie, et d'une ouverture de remplissage, l'autre chambre étant remplie par un agent propulseur, qui se dilate de façon isobare en se vaporisant, pour la délivrance de la substance à délivrer, caractérisé en ce que le septum (10) sert à fermer de façon étanche la chambre (9), qui contient la substance devant être délivrée.

3. Dispositif équipé d'un septum selon la revendication 1, pour la délivrance dosée d'une substance, par exemple d'un médicament, le dispositif possédant deux chambres séparées l'une de l'autre par une membrane flexible et dont l'une, qui contient la substance devant être délivrée, est pourvue d'une ouverture de sortie raccordée à un cathéter de sortie, et d'une ouverture de remplissage, l'autre chambre étant remplie par un agent propulseur, qui se dilate de façon isobare en se vaporisant, pour la délivrance de la substance à délivrer, et dans lequel un dispositif pour une introduction supplémentaire de médicaments ou analogues dans le cathéter de sortie est prévu entre l'ouverture de délivrance de la chambre et le cathéter de sortie, caractérisé en ce que le septum annulaire (10) sert à fermer de façon étanche la chambre (9) pour l'introduction supplémentaire de médicaments, le septum étant disposé autour de l'ouverture de remplissage (12).

4. Dispositif équipé d'un septum selon la revendication 3, caractérisé en ce que l'ouverture de remplissage (12) est prévue en position centrée dans le dispositif et que le septum annulaire (10) est disposé concentriquement par rapport à l'ouverture de remplissage (12).
